# EUROPEAN PATENT APPLICATION

(11) **EP 2 272 564 A1**
(43) Date of publication of application: **12.01.2011**
(21) Application number: 10159784.7
(22) Date of filing: 13.04.2010
(51) Int. Cl.: A61N 5/10, A61B 6/02

(54) **Digital tomosynthesis in ion beam therapy systems**

(30) Priority: 09.07.2009 US 500521
(71) Applicant: Siemens Aktiengesellschaft, 80333 München (DE)
(72) Inventor: Bani-Hashemi, Ali-Reza, Walnut Creek, 94595 (US); Maltz, Jonathan S., Oakland, 94611 (US)

(57) **Abstract**

Some embodiments include creation (S201) of projection images of a patient volume using one or more x-ray sources, performance (S202) of digital tomosynthesis on the projection images to generate a cross-sectional image of the patient volume, determination (S203) of a location of a target within the patient volume based on the cross-sectional image, determination (S204) of a location of a Bragg peak associated with an ion beam, and determination (S205) of a difference between the location of the target and the location of the Bragg peak.

In some aspects, the patient volume is moved to move the target toward the location of the Bragg peak based on the difference. Additionally or alternatively, one or more characteristics of the ion beam may be changed to move the location of the Bragg peak toward the location of the target based on the difference. The one or more radiation sources may comprise a plurality of x-ray sources disposed in a fixed relationship to each other. Each of the plurality of x-ray sources may also be disposed in a fixed relationship to the ion beam source.

## Description

### BACKGROUND

### Field

The embodiments described below relate generally to systems for delivering ion beam therapy. More specifically, some embodiments are directed to treatment verification systems used in conjunction with such delivery.

### Description

Conventional radiation therapy systems direct a beam of protons, x-ray photons, neutrons or ions to a target within a patient volume. The particles kill tissue cells within the target by damaging the DNA thereof. The dose delivered by an x-ray beam, for example, exhibits a maximum at shallow tissue depths and decays exponentially as the depth increases.

Imaging systems are conventionally used to ensure that a target is located in the path of an x-ray beam and that sensitive structures are avoided in accordance with a treatment plan. Such imaging systems may therefore acquire projection images along the axis of the x-ray beam in order to determine whether internal structures are located in the beam path. Specifically, the "beam's eye" view provided by such projection images identifies the relationship between the beam path and the internal structures, but only in directions perpendicular to the beam axis. This limitation is acceptable in x-ray treatment, due to the gradual decline of dose with increasing depth.

For therapeutic ion beams, the delivered dose typically increases with increasing tissue depth, up to a depth known as the Bragg peak. At depths greater than the Bragg peak, the dose drops to zero (for protons) or almost zero (for heavier ions). The location of the Bragg peak depends on factors including, but not limited to, the type of ion, the energy of the ion beam and the type of intervening tissue.

The present inventors have recognized that conventional inline imaging as described above is insufficient for image guidance in ion beam delivery systems. Particularly, small changes in the location of a target along an ion beam axis may result in large changes in the dose delivered to the target. Since conventional inline imaging is unable to efficiently detect such changes in location, other systems to provide image guidance for ion beam therapy are desired.

### SUMMARY

In order to address the foregoing, some embodiments provide creation of projection images of a patient volume using one or more x-ray sources, performance of digital tomosynthesis on the projection images to generate a cross-sectional image of the patient volume, determination of a location of a target within the patient volume based on the cross-sectional image, determination of a location of a Bragg peak associated with an ion beam, and determination of a difference between the location of the target and the location of the Bragg peak.

According to some aspects, the patient volume is moved to move the target toward the location of the Bragg peak based on the difference. Additionally or alternatively, one or more characteristics of the ion beam may be changed to move the location of the Bragg peak toward the location of the target based on the difference.

Some aspects provide delivery of the ion beam during creation of the projection images of the patient volume, performance of digital tomosynthesis on the projection images, determination of the location of the target within the patient volume, and determination of the difference.

The one or more radiation sources may comprise a plurality of x-ray sources disposed in a fixed relationship to each other. Each of the plurality of x-ray sources may also be disposed in a fixed relationship to the ion beam source.

The appended claims are not limited to the disclosed embodiments, however, as those in the art can readily adapt the descriptions herein to create other embodiments and applications.

### BRIEF DESCRIPTION OF THE DRAWINGS

Embodiments will become readily apparent from consideration of the following specification as illustrated in the accompanying drawings, in which like reference numerals designate like parts, and wherein:
FIG. 1 is a perspective view of a treatment room according to some embodiments;
FIG. 2 is a flow diagram of process steps pursuant to some embodiments;
FIG. 3 is a perspective view of a treatment room according to some embodiments;
FIG. 4 is a perspective view of a treatment room according to some embodiments;
FIG. 5 is a perspective view of a treatment room according to some embodiments; and
FIG. 6 is a perspective view of a treatment room according to some embodiments.
FIG. 7 is a perspective view of a treatment room according to some embodiments.

### DETAILED DESCRIPTION

The following description is provided to enable a person in the art to make and use some embodiments and sets forth the best mode contemplated by the inventors for carrying out some embodiments. Various modifications, however, will remain readily apparent to those in the art.

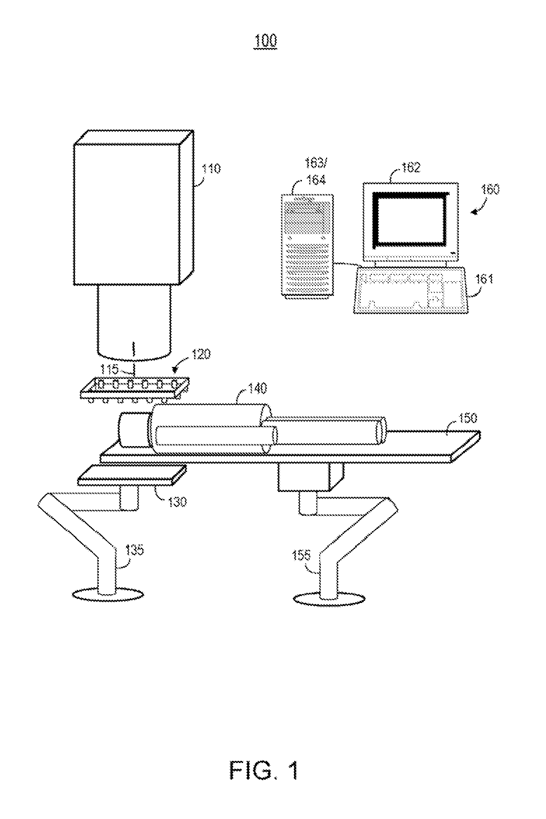

FIG. 1 is a perspective view of treatment room 100 according to some embodiments. Located within treatment room 100 are ion beam source 110, x-ray sources 120, detector 130, patient 140, table 150 and operator console 160. Detector 130 and table 150 are coupled to robotic arms 135 and 155, respectively, to provide three-dimensional movement thereof. The elements of treatment room 100 may be used in some embodiments to verify a target position and to deliver ion beam therapy. More particularly, some embodiments comprise performance of digital tomosynthesis to determine a location of a target with respect to a location of a Bragg peak and to co-locate the target and the Bragg peak if necessary.

Beam source 110 may comprise any source of proton, neutron, or heavier ion beams that is or becomes known. Generally, ion beam source 110 may comprise a linear accelerator coupled to a synchrotron to accelerate a beam of ions to a desired energy (e.g., 70 to 250 MeV). Ion beam source 110 may also include a scanning system to deflect the ion beam in two dimensions according to a treatment plan. Accordingly, the deflections may be controlled so as to scan the ion beam across a downstream two-dimensional area (i.e., a cross-section of the target).

Ions exit ion beam source 110 along beam axis 115, which may change direction if the beam is deflected as described above. The energies with which the ions exit ion beam source 110 determine the location of maximum energy deposition (i.e., the Bragg peak) associated with the ion beam. By varying the energies and employing the above-mentioned scanning method, the Bragg peak can be moved throughout a target in three dimensions.

X-ray sources 120 may comprise any sources to emit kilovoltage radiation or other imaging radiation that are or become known. In some embodiments, x-ray sources 120 employ cathodes based on carbon nanotube or thermionic emission technology. X-ray sources 120 are affixed to support 125 such that each x-ray source 120 is disposed in a fixed relationship to each other x-ray source 120. Moreover, in some embodiments, each x-ray source 120 is disposed in a fixed relationship with respect to ion beam source 110. Support 125 may be mounted to source 110 and/or to an unshown support. The unshown support, such as a multi-jointed robotic arm, may move support 125 (and x-ray sources 120) in three dimensions and rotate support 125 around one or more rotational axes.

X-ray sources 120 may comprise any of the arrangements and operate in any of the manners described in commonly-assigned co-pending applications (Attorney docket nos. 2007P010274US and 2008P00307US), and in Fixed Gantry Tomosynthesis System For Radiation Therapy Image Guidance Based On A Multiple Source X-Ray Tube With Carbon Nanotube Cathodes, Maltz et al., Med. Phys. 36 (5), May 2009, pp. 1624-1636.

Detector 130 may acquire projection images before, during and/or after radiation treatment. For example, detector 130 may be used to acquire projection images for verification and recordation of a target position and of an internal patient portal to which radiation is delivered. Detector 130 may comprise any system or systems to acquire an image based on received radiation.

In some embodiments, detector 130 is a flat-panel imaging device using a scintillator layer and solid-state amorphous silicon photodiodes deployed in a two-dimensional array. In operation, the scintillator layer receives photons and generates light in proportion to the intensity of the received photons. The array of photodiodes receives the light and records the intensity of received light as stored electrical charge. The stored charge therefore comprises an acquired image that represents intensities at each location of a radiation field produced by a radiation beam. The bounds of the radiation field are determined by the physical intersection of the radiation beam with the surface of the scintillator layer.

Detector 130 may comprise other types of imaging devices. For example, incoming radiation may also be converted to and stored as electrical charge without use of a scintillator layer. In such imaging devices, radiation is absorbed directly by an array of amorphous selenium photoconductors. The photoconductors convert the radiation directly to stored electrical charge that comprises an acquired image of a radiation field.

Detector support 135 may comprise a multi-jointed robotic arm to move detector 130 to a desired position with a desired orientation. In some embodiments, support 135 may be coupled to move detector 130 via a ball joint or other type of coupling to provide roll, pitch and yaw movement of detector 130 with respect to support 135. Examples of a multi-jointed robotic arm include, but are not limited to, the robotic arm used in the Cyberknife® system by Accuray®.

In one example of operation, patient 140 is placed on table 150 and one or more of x-ray sources 120, detector 130 and table 150 are moved such that detector 130 is parallel to and facing x-ray sources 120 with a volume of patient 140 positioned therebetween. Such a configuration is illustrated in FIG. 1, where x-ray sources 120 and detector 130 are also aligned with beam axis 115.

Each of x-ray sources 120 successively emits radiation toward detector 130. Detector 130 acquires a projection image based on each successive emission. More particularly, detector 130 develops and stores charge representing radiation intensities at each location of a radiation field produced by each successive emission. Since the patient is located between sources 120 and detector 130, the radiation intensity at a particular location represents the attenuative properties of tissues along a divergent line between the emitting source 120 and the particular location. A set of radiation intensities acquired by detector 130 in response to a particular emission may therefore comprise a two-dimensional projection image of these tissues.

Each projection image, taken alone, is of limited use in resolving positions of internal structures of the patient volume, particularly their depth along beam axis 115. Processor 163 of computer system 160 therefore performs digital tomosynthesis on the projection images to generate a cross-sectional image of the patient volume using known techniques. The cross-sectional image represents a particular plane of the patient viewed from a particular perspective (e.g., along beam axis 115).

Operator console 160 may be in communication with one or more of the above-described elements of FIG. 1 to provide control and/or monitoring thereof. Operator console 160 includes input device 161 for receiving instructions from an operator and output device 162, which may be a monitor for presenting beam characteristics of ion beam source 110, projection images acquired by detector 130, computed tomography images used for treatment planning, Bragg peak locations, cross-sectional digital tomosynthesis images, interfaces for receiving operator instructions, and/or operator alerts. According to some embodiments, output device 162 may present an alert notifying an operator of a positioning error prior to or during treatment delivery.

Input device 161 and output device 162 are coupled to processor 163 and storage 164. Processor 163 may execute program code to perform or cause to be performed any of the determinations and generations described herein. For example, processor 133 may perform digital tomosynthesis on projection images acquired by detector 130 to generate a cross-sectional image of a patient volume viewed from a perspective of ion beam source 110.

A hardware environment according to some embodiments may include less or more elements than those shown in FIG. 1. For example, unseen elements may be required to provide AC power, RF power, cooling, hydraulics, vacuum and/or other systems needed to operate the illustrated elements. In addition, embodiments are not limited to the illustrated elements and/or environment.

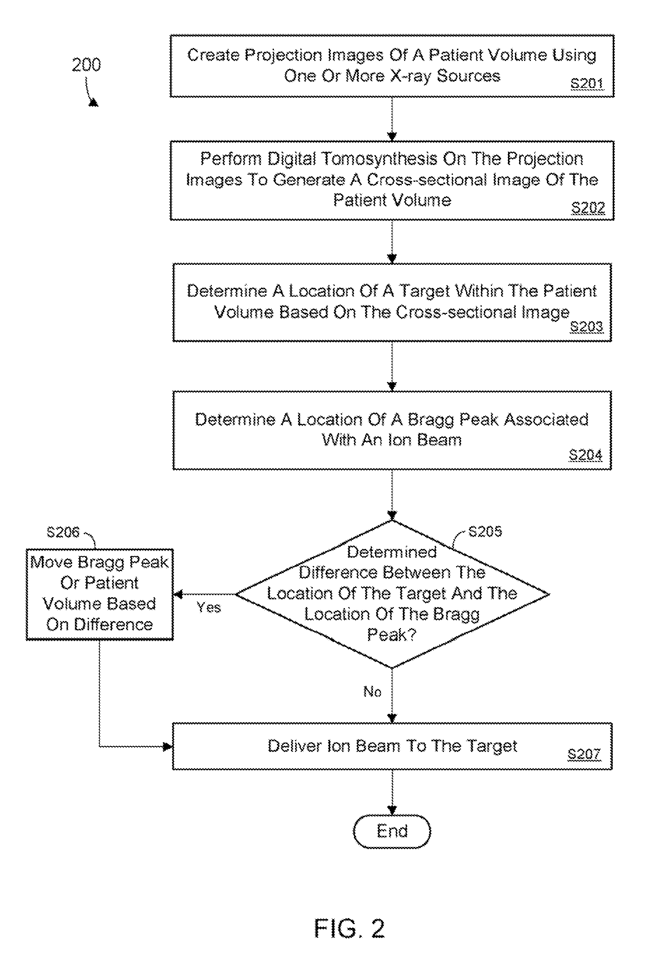

FIG. 2 is a flow diagram of a process according to some embodiments. Process 200 and the other processes described herein may be performed using any suitable combination of hardware, software or manual means. Software embodying these processes may be stored by any tangible medium, including but not limited to a fixed disk, a floppy disk, a CD-ROM, a DVD-ROM, a Zip™ disk, and a magnetic tape. Examples of these processes will be described below with respect to the elements of treatment room 100, but embodiments are not limited thereto.

Process 200 may be performed after a patient has been placed on a treatment table and is awaiting ion therapy. Process 200 may be preceded any suitable quality assurance procedures. In some embodiments, process 200 is preceded by acquisition of correction images for performing corrections on images acquired by detector 120. These corrections may comprise offset correction to account for dark current effects, gain correction to account for variations in pixel sensitivity, and dead pixel correction to account for malfunctioning pixels.

A treatment plan is also established prior to process 200. The treatment plan may define multiple treatment fractions, each of which includes one or more discrete beams to be delivered. For each beam, the treatment plan may specify characteristics (e.g., energy, type, etc.) of the beam, location of the Bragg peak along beam axis 115 and a cross-sectional image of a target at the Bragg peak associated with the beam and orthogonal to the beam axis. The cross-sectional image may be generated based on a computed tomography image used to create the treatment plan. As described above, each beam can be static or moving, and/or may exhibit multiple radiation shapes delivered discretely (i.e., segments or ports) or continuously.

Initially, at S201, projection images of a patient volume are created using one or more x-ray sources. For example, radiation may be successively emitted from each one of x-ray sources 120 and detector 130 may develop, store and output projection images based on the emitted radiation. As described above, each of the projection images may comprise a two-dimensional set of radiation intensities representing the attenuative properties of tissues between an x-ray source 120 and detector 130.

The projection images acquired at S201 may be corrected or transformed based on characteristics of detector 130 and/or based on the fixed relationship between x-ray sources 120. In the latter regard, the projection images may be modified to account for the different distances over which different portions of the emitted radiation travel to reach detector 130.

Digital tomosynthesis is performed on the projection images at S202 to generate a cross-sectional image of the patient volume. The cross-sectional image may represent a "slice" of the patient volume that is orthogonal to beam axis 115 of ion beam source 110.

According to some embodiments, x-ray sources 120 may be disposed in a circular configuration, an elliptical configuration, a triangular configuration, a square configuration, a rectilinear configuration (i.e., as illustrated), and/or along the sides of any polygon. Digital tomosynthesis processing in conjunction with such configurations may include an iterative reconstruction method such as the algebraic reconstruction technique (ART), the simultaneous algebraic reconstruction technique (SART), the simultaneous iterative reconstruction technique (SIRT) or the ordered subsets expectation maximization (OSEM) algorithm. More generally, any methods that solve for the values of the voxels within the imaged field-of-view based on the measured projection values are suitable for tomosynthesis reconstruction. Such methods include techniques of optimization and/or regression, and may be applied to maximize certain characteristics (e.g., statistical likelihood, statistical entropy) and/or to minimize others (e.g., the sum of squared residuals).

A location of a target within the patient volume is determined at S203 based on the cross-sectional image. For example, using the configuration shown in FIG. 1, one or more tomosynthesis-generated cross-sectional images are analyzed to determine depths corresponding to one or more cross-sections of the target. In comparison to two-dimensional projection images alone, digital tomosynthesis provides improved delineation of depth-resolved tissue boundaries due to the reduced influence of under-and overlying structures. Accordingly, if the geometry of projection image acquisition corresponds to the geometry of beam delivery, the generated cross-sectional image may be used to efficiently determine a depth of internal structures along the beam axis.

A location of a Bragg peak associated with an ion beam to be delivered is determined at S204. The location of the Bragg peak may be determined mathematically based on a priori knowledge of the beam's characteristics and other known factors. In some embodiments, the location of the Bragg peak is observed through imaging.

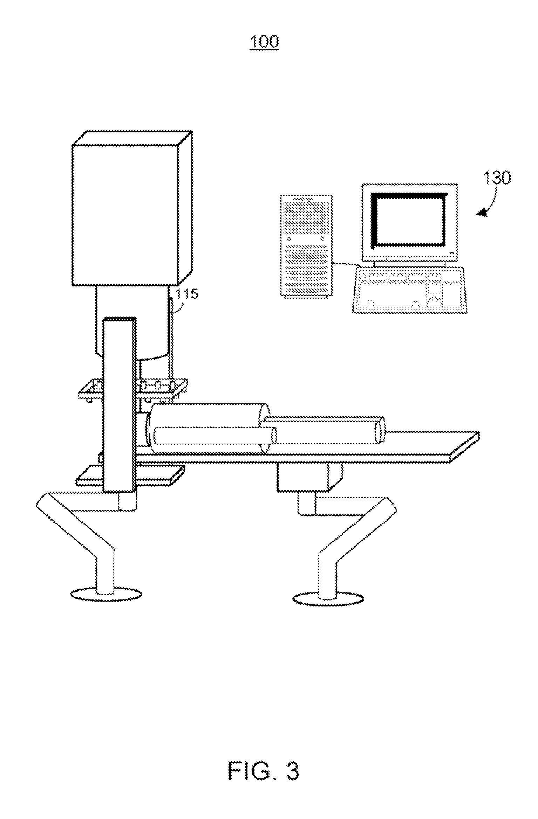

FIG. 3 is a perspective view of treatment room 300 including positron emission tomography imager 310. Imager 310 may determine the location of a Bragg peak associated with a delivered ion beam by detecting positron-electron annihilation events resulting from interaction with the ion beam with patient 140. Any type of system for determining the location of positron-electron annihilation events may be used at S204 in some embodiments.

Next, at S205, any differences between the location of the target and the location of the Bragg peak are determined. In some embodiments, S203 through S205 comprise determining whether an actual cross-sectional image of the target at the location of the Bragg peak conforms to a cross-sectional image of the target expected by the treatment plan. If the actual image fails to conform to the expected image by a measure greater than a threshold, flow proceeds to S206. In some embodiments, actual cross-sectional images of the target at depths other than the Bragg peak are also compared to expected cross-sectional images during the determination at S205.

The Bragg peak or the patient volume, or both are moved at S206 based on the difference determined at S205. The Bragg peak may be moved by changing characteristics of the ion beam (e.g., energy) and the patient volume may be moved by moving table 150 and/or repositioning patient 140 on table 150. The ion beam is delivered to the target at S207.

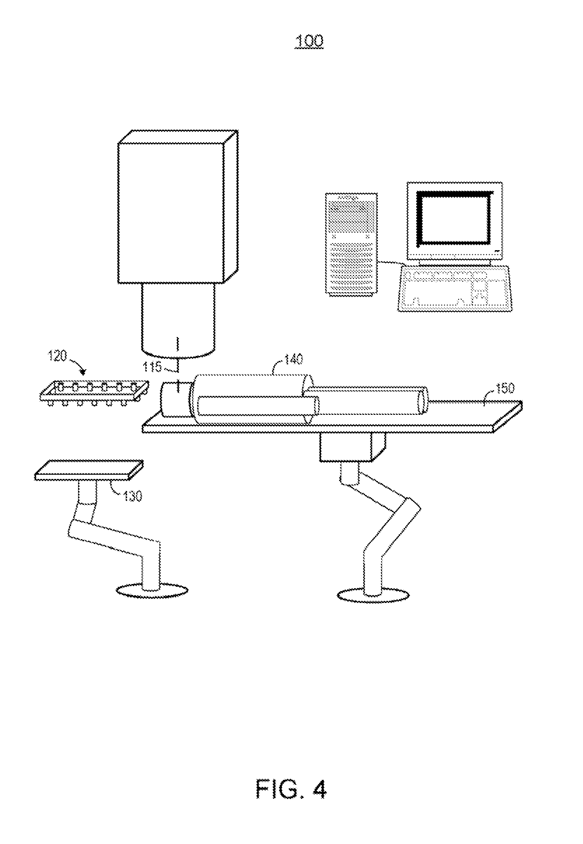

FIG. 4 illustrates treatment room 100 of FIG. 1 during beam delivery according to the present example. Table 150 has been moved the change the position of patient 140 relative to beam axis 115, and detector 130 and x-ray sources 120 have been moved out of the way to prevent damage thereto. In some embodiments, one or both of detector 130 and x-ray sources 120 may remain in their respective image acquisition position during beam delivery.

In some embodiments, flow returns to S202 after S206 to check whether the movement(s) performed in S206 resulted in conformity of the target and the Bragg peak as desired by the treatment plan. If it is determined at S205 that the difference between the location of the Bragg Peak and the location of the target is less than a threshold, flow proceeds to S207 for delivery of the ion beam according to the currently-determined beam characteristics.

According to some embodiments, flow returns to S202 after S207 and proceeds as described above during delivery of the ion beam. Such an arrangement may provide monitoring of the relative positions of the target and the Bragg peak during beam delivery. Such an arrangement may be particularly suited for determination of the Bragg peak using an imager as shown in FIG. 3 due to the annihilation events caused by beam delivery. Delivery of the beam in these embodiments may terminate if flow proceeds from S205 to S206 as also described above.

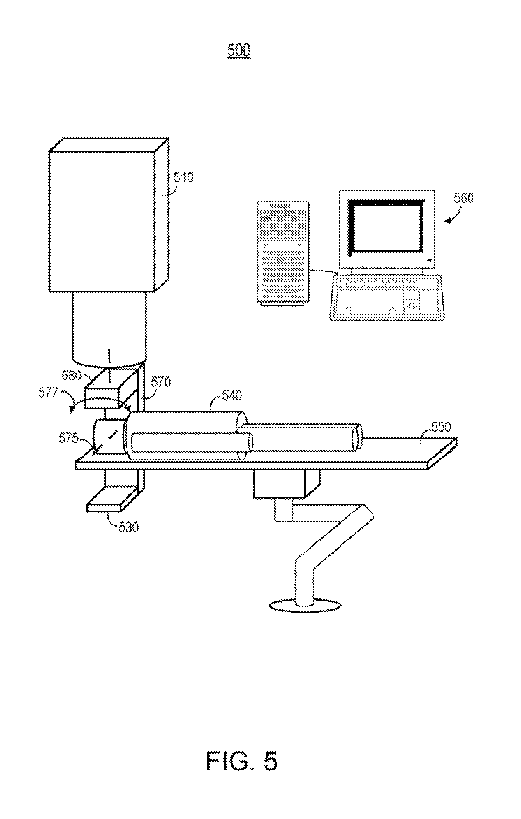

FIG. 5 illustrates treatment room 500 according to some embodiments. Elements 510, 530, 540, 550 and 560 may be embodied as described with respect to similarly-numbered elements of FIG. 1. However, detector 530 is mounted to C-arm 570.

X-ray source 580 is also mounted to C-arm 570. X-ray source 580 may comprise any of the x-ray sources mentioned above and/or a conventional x-ray tube. C-arm 570 rotates around axis 575 as illustrated by arrow 577, thereby rotating detector 530 and x-ray source 580 around axis 575. This rotation, and the fixed relationship between detector 530 and x-ray source 580, provide for creation of projection images according to some implementations of S201.

Unlike the projection images created using the system of FIG. 1, these projection images are created by emitting radiation along an arc subtended from a common point. Accordingly, creation of the cross-sectional image at S202 may require digital tomosynthesis reconstruction algorithms different from those described above, such as filtered back-projection algorithms. For example, the projection images may be filtered with a Ram-Lak filter before back-projection.

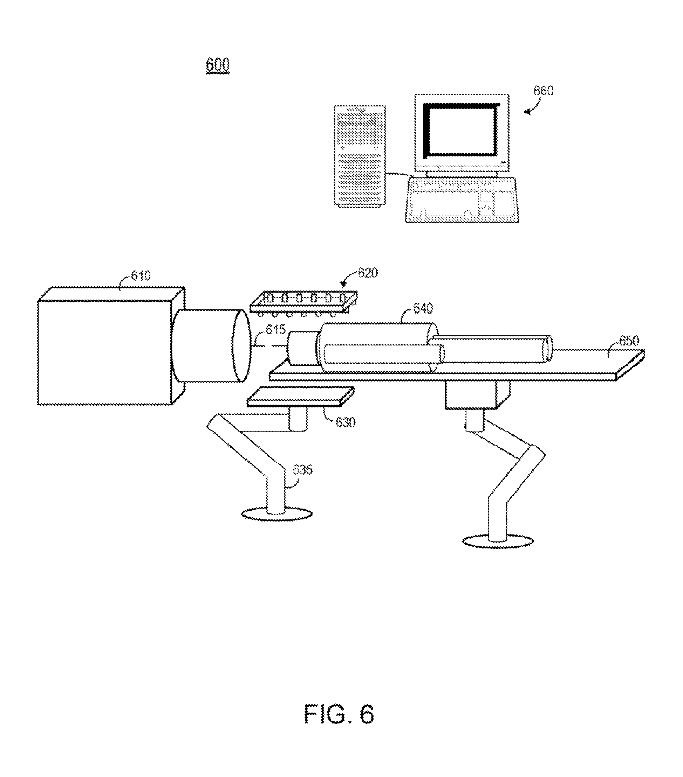

FIG. 6 illustrates treatment room 600 according to some embodiments. Each illustrated element may be embodied as described with respect to similarly-numbered elements of FIG. 1. In contrast to FIG. 1, however, the plane of x-ray sources 620 is parallel to beam axis 615. Accordingly, projection images created at S201 using the FIG. 6 configuration exhibit a different perspective than those created by the FIG. 1 configuration. Some embodiments substitute a C-arm or other single-source configuration such as that shown in FIG. 5 to create projection images from the different perspective.

Using the FIG. 6 implementation, the tomosynthesis-generated cross-sectional images parallel to the plane of x-ray sources 620 will provide the best depth resolution and are therefore analyzed to determine the location of the target within the patient volume at S203. In particular, the location of the target is determined with reference to the cross-sectional images located at the depths through which the beam will pass.

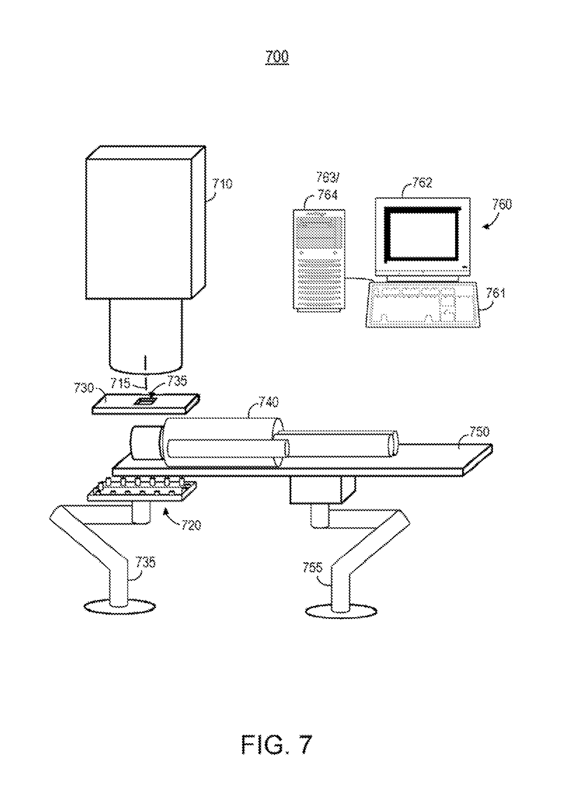

FIG. 7 illustrates treatment room 700 according to some embodiments. Each illustrated element may be embodied as described with respect to similarly-numbered elements of FIG. 1. The elements of treatment room 700 may perform process 200 according to some embodiments.

In contrast to FIG. 1, detector 730 is located between ion beam source 710 and table 750. Detector 730 also defines hole 735 through which a delivered ion beam may pass. According to some embodiments, detector 730 comprises two or more separate detectors tiled around beam axis 715.

The embodiments described herein show a patient volume. However, embodiments are not restricted to patient volumes. Volumes of inanimate bodies such as phantoms or calibration equipment may be used in connection with embodiments, for example for research purposes or during commissioning or quality control of a system.

The several embodiments described herein are solely for the purpose of illustration. Therefore, persons in the art will recognize from this description that other embodiments may be practiced with various modifications and alterations.

## Claims

1. A method comprising:
creating (S201) projection images of a patient volume using one or more x-ray sources;
performing (S202) digital tomosynthesis on the projection images to generate a cross-sectional image of the patient volume;
determining (S203) a location of a target within the patient volume based on the cross-sectional image;
determining (S204) a location of a Bragg peak associated with an ion beam; and
determining (S205) a difference between the location of the target and the location of the Bragg peak.

2. A method according to Claim 1, further comprising:
moving (S206) the patient volume to move the target toward the location of the Bragg peak based on the difference.

3. A method according to Claim 1, further comprising:
changing (S206) one or more characteristics of the ion beam to move the location of the Bragg peak toward the location of the target based on the difference.

4. A method according to Claim 1, further comprising:
delivering (S207) the ion beam to the target.

5. A method according to Claim 4, wherein the ion beam is delivered during creation of the projection images of the patient volume, performance of digital tomosynthesis on the projection images, determination of the location of the target within the patient volume, and determination of the difference.

6. A method according to Claim 1, wherein determining the location of the Bragg peak comprises:
calculating the location of the Bragg peak based on characteristics of the ion beam.

7. A method according to Claim 1, wherein determining the location of the Bragg peak comprises:
acquiring positron emission tomography images of the patient volume; and
determining the location of the Bragg peak based on the positron emission tomography images.

8. A system comprising:
an ion beam source (110);
one or more x-ray sources (120);
a detector (130) to acquire projection images of a patient volume based on x-rays emitted from one or more of the one or more x-ray sources; and
a processor (163) to:
perform digital tomosynthesis on the projection images to generate a cross-sectional image of the patient volume;
determine a location of a target within the patient volume based on the cross-sectional image;
determine a location of a Bragg peak associated with an ion beam associated with the ion beam source; and
determine a difference between the location of the target and the location of the Bragg peak.

9. A system according to Claim 8, wherein the one or more x-ray sources comprise:
a plurality of x-ray sources disposed in a fixed relationship to each other.

10. A system according to Claim 9, wherein each of the plurality of x-ray sources is disposed in a fixed relationship to the ion beam source.

11. A system according to Claim 8, further comprising:
a table (150) to move the patient volume with respect to the ion beam source so as to move the target toward the location of the Bragg peak based on the difference.

12. A system according to Claim 8, the processor further to:
change one or more characteristics of the ion beam associated with the ion beam source to move the location of the Bragg peak toward the location of the target based on the difference.

13. A system according to Claim 8, the ion beam source to deliver the ion beam to the target.

14. A system according to Claim 13, wherein the ion beam source delivers the ion beam during creation of the projection images of the patient volume, performance of digital tomosynthesis on the projection images, determination of the location of the target within the patient volume, and determination of the difference.

15. A system according to Claim 8, wherein the processor is to determine the location of the Bragg peak by calculating the location of the Bragg peak based on characteristics of the ion beam.

16. A system according to Claim 8, further comprising:
a positron emission tomography imager (310) to acquire positron emission tomography images of the patient volume,
wherein the processor is to determine the location of the Bragg peak based on the positron emission tomography images.
